# EUROPÄISCHE PATENTSCHRIFT

(11) **EP 1 216 028 B1**
(45) Veröffentlichungstag und Bekanntmachung des Hinweises auf die Patenterteilung: **27.12.2006**
(21) Anmeldenummer: 00964255.4
(22) Anmeldetag: 29.09.2000
(51) Int. Cl.: A61K 9/16

(54) **BIOABBAUBARE TRÄGERSYSTEME FÜR THERAPEUTISCH WIRKSAME SUBSTANZEN UND VERFAHREN ZU DEREN HERSTELLUNG**
BIODEGRADABLE EXCIPIENT SYSTEMS FOR THERAPEUTICALLY ACTIVE SUBSTANCES AND METHOD FOR PRODUCING THE SAME
SYSTEMES D'EXCIPIENTS BIODEGRADABLES POUR SUBSTANCES A EFFET THERAPEUTIQUES ET PROCEDE DE PRODUCTION DESDITS SYSTEMES

(30) Priorität: 01.10.1999 DE 19947354
(43) Veröffentlichungstag der Anmeldung: 26.06.2002
(73) Patentinhaber: Glatt Process Technology GmbH, 79589 Binzen (DE)
(72) Erfinder: PRASCH, Armin, 79100 Freiburg (DE); LUY, Bernhard, 79102 Freiburg (DE)
(74) Vertreter: Pfenning, Meinig & Partner GbR
(86) Internationale Anmeldenummer: PCT/EP2000/009558
(87) Internationale Veröffentlichungsnummer: WO 2001/024776

(56) Entgegenhaltungen:
- EP-A- 0 166 263
- EP-A- 0 576 675
- WO-A-96/32149
- WO-A-97/44015
- WO-A-98/31346
- WO-A1-00/24436
- WO-A1-00/38752
- DE-A- 3 517 456
- DE-C- 4 441 167
- DE-C- 19 849 589
- VOIGT R: "Lehrbuch der pharmaceutischen Technologie" 1984, VERLAG CHEMIE , WEINHEIM

## Beschreibung

Die Erfindung betrifft eine geeignete Formulierung eines bioabbaubaren Trägersystems für therapeutisch wirksame, pharmazeutische Substanzen, die einerseits die Wundheilung fördern oder die gezielt zusätzliche pharmakologische Wirkungen im Organismus bewirken können. Die Freigabe des Wirkstoffs soll bei den beschriebenen Arzneiformen verzögert und allmählich erfolgen, wodurch für diese Arzneiformen eine verlängerte Wirkung im Sinne eines Depots erzielt wird. Bei den Trägersystemen handelt es sich um bioabbaubare Polymere, die im Human- oder Veterinär-Organismus toxikologisch unbedenklich, verträglich und immunogen sind. Erfindungsgemäß werden Blutplasmaproteine, insbesondere Fibrinkleber-Komponenten Fibrinogen und Thrombin eingesetzt. Die Herstellung erfolgt durch ein Granulations- bzw. Sprühagglomerationsverfahren in einer Wirbelschicht, wodurch spezifische Produkteigenschaften eingestellt werden können.

Fibrinkleber oder Gewebekleber werden in der Human-Medizin und in bestimmten Fällen (z.B. Rennpferde) auch in der Veterinär-Medizin in der Regel z.B. bei chirurgischen Eingriffen zur Förderung der Blutgerinnung bzw. Blutstillung und zum Verschließen von Wunden eingesetzt. Das Prinzip der Fibrinklebung entspricht der letzten Stufe des natürlichen Blutstillungssystems (Hämostase) bei Säugern, einer Co-Enzym/Enzym gesteuerten Kaskadenreaktion, bei der Fibrinogen durch Thrombin in Anwesenheit von Faktor XIII und Ca2+-Ionen zu Fibrin umgesetzt wird. Während der Wundheilung wird das Fibrin wieder proteolytisch abgebaut und dadurch auf natürlichem Weg absorbiert. Technisch gleicht das Funktionsprinzip der Fibrinkleber dem Prinzip von Zwei- bzw. Mehrkomponentenklebern, die in der Regel entweder erst an der zu klebenden Stelle oder auch nur kurz vor dem eigentlichen Zeitpunkt miteinander gemischt bzw. in Kontakt gebracht werden.

Bei der Darreichung und Anwendung eines Fibrin-Gewebeklebers ist darauf zu achten, daß Fibrinogen und Thrombin erst direkt am Ort der Blutung zusammengebraucht werden, da die Gerinnung spontan einsetzt. Benachbarte Stellen sind aufgrund der sehr guten Haftwirkung dabei gut abzudecken. Voraussetzung für die Gerinnung ist die freie Beweglichkeit der einzelnen beteiligten Moleküle z.B. in Wasser. Praktisch wird dies in der Regel so gelöst, daß die beiden entscheidenden Komponenten Fibrinogen und Thrombin bis zur Applikation an der Wunde getrennt separiert werden und erst direkt an der Wunde in Kontakt miteinander gebracht werden.

Die Komponenten müssen jeweils steril verpackt werden und in einer geeigneten Form und unter definierten Bedingungen so aufbewahrt werden, daß die Aktivität der einzelnen Proteine bzw. Enzyme durch die Lagerung nicht geschädigt wird. In der Regel wird dies so gelöst, daß die Proteinkonzentrate in gefriergetrockneter Form in Vials vorliegen. In dieser Form sind sie bei Kühlschrankbedingungen (4 bis 8 °C) für eine bestimmte Zeit und für eine kürzere Zeit auch bei Raumbedingungen (20 °C) lagerstabil. Gefriergetrocknet liegt das Konzentrat jedoch in fester, komprimierter und dadurch unbeweglicher Form, jedoch als löslicher Feststoff vor. Deshalb müssen die Proteinkonzentrate vor der Anwendung wieder vollständig in Lösung gebracht werden, um die gewünschte biochemische Reaktion starten zu können. Alternativ dazu können die Komponenten auch in tiefgefrorener, fester Form lagerstabil gehalten werden, die dann vor der Anwendung aufgetaut werden müssen und getrennt als Lösung zur Applikation kommen.

Die beiden Lösungen können dann jeweils über Injektionsspritzen z.B. im gleichen Volumenverhältnis zugegeben werden. Dabei ist die Fibrinogen-Lösung zuerst auf die Wunde aufzubringen und möglichst sofort mit der Thrombin-Lösung zu überschichten. Die zu klebenden Teile sind dann so lange zu fixieren, bis eine vorläufige Verfestigung eingetreten ist.

Weiterhin wird in der Patentanmeldung WO 97/44015 die Herstellung von Mikropartikeln auf Fibrinogen- und Thombinbasis beschrieben, die jeweils einzeln sprühgetrocknet werden. Der damkt verbundenen Wasserentzug setzt die Beweglichkeit der Proteinmoleküle soweit herab, daß die Gerinnung nicht spontan einsetzen kann. Die Mikropartikel sind alle kleiner 20 µm, bevorzugt kleiner 10 µm bzw. 2-5 µm, und sollen gut löslich sein. Miteinander vermischt können diese Fibrinogen- und Thrombin-haltigen Mikropartikel zur Blutstillung eingesetzt werden. Ein Nachteil ist jedoch, daß es sich dabei um ein stark staubendes Pulver handelt, wodurch eine direkte Applikation praktisch nicht möglich ist.

Kennzeichen der Fibrinkleber humanen, tierischen oder auch rekombinanten Ursprungs sind die sofortige und vergleichsweise starke Adhäsion des gebildeten Fibrins an dem Ort der Aufbringung (z.B. Wunde, Gewebe), die sich im vernetzten Fibrin ausbildende Matrixstruktur und die selbständige, biologische Abbaubarkeit des Fibrins. Weiterhin zeichnen sich die natürlichen Komponenten dadurch aus, daß diese Komponenten bereits im menschlichen oder auch tierischen Organismus vorhanden sind und deshalb toxikologisch unbedenklich und gut verträglich sind.

Aufgrund dieser Kennzeichen (Adhäsion, Matrix-Struktur, Verträglichkeit und Abbaubarkeit) können Fibrinkleber bzw. Komponenten eines Fibrinklebers ein geeignetes Trägersystem für zusätzliche therapeutisch wirksame Substanzen sein. Da ein Fibrinkleber jedoch wie beschrieben nur als Lösung bzw. in 2 getrennten Lösungen appliziert werden kann, ist es problematisch, für wirkstoffhaltige Firbinkleber-Formulierungen homogene freisetzungskontrollierende Matrix-Strukturen aufzubauen.

Aus einer Reihe von Veröffentlichungen sind Fibrinkleber als Träger für Wirkstoffe bereits bekannt. Neben dem Einsatz von Antibiotika zur Unterdrückung von lokalen Infektionen wurden auch Zytostatika Fibrinklebern zugegeben, um z.B. verbleibende Krebszellen nach der operativen Entfernung des Primärtumors lokal chemo-therapeutisch zu behandeln. Auch wurde z.B. Zink in Fibrinkleber mit eingearbeitet, um so einen höheren Gehalt an Zink über einen längeren Zeitraum direkt in der Wunde zu erzielen und so eine verbesserte Wundheilung zu ermöglichen (US 5,651,982). Weiterhin wird auch in EP 804153A1 die Kombination eines Fibrinklebers mit einem therapeutischen Wirkstoff beschrieben, der z.B. nach der operativen Entfernung eines Tumors als radiotherapeutischer Wirkstoff eingesetzt werden kann.

Somit ist bekannt, daß Fibrinkleber als Trägersystem für therapeutische Wirkstoffe eingesetzt werden können. Durch geeignete Maßnahmen kann die Wirkstofffreisetzung so kontrolliert werden, daß die Freisetzung verzögert über einen bestimmten Zeitraum erfolgt. Die Freisetzung dieser Wirkstoffe aus dem Trägersystem Fibrinkleber variiert gemäß den Angaben in der wissenschaftlichen Literatur von 24 Stunden bis einigen Tagen; bei schwer löslichen Wirkstoffen kann die Freisetzung sogar bis zu 40 Tagen dauern. Demnach ist es möglich, mittels der Kombination eines Fibrinklebers mit therapeutisch wirksamen Substanzen auch eine verzögerte bzw. verlängerte Wirkstofffreisetzung mit einer langsamen, konstanten Wirkstoffaufnahme in den Blutkreislauf und dadurch eine konstante Blutspiegelkonzentration des Wirkstoffs zu erzielen.

Generell sind derartige Darreichungsformen unter dem Begriff Depotarzneiformen bekannt und Ziel zahlreicher Entwicklungen. Depotarzneiformen werden auch parenteral angewandt. Vorteilhaft ergibt sich dabei z.B., daß statt einer i.v. Dauerinfusion Patienten Depotarzneiformen mit verzögerter Freisetzung verabreicht werden können, was für den Patienten eine erheblich höhere Selbständigkeit und Beweglichkeit bedeutet. Dies kann auch ermöglichen, durch eine gezielte lokale Applikation und eine sich daraus ergebende lokale Wirkstofffreisetzung, z.B. hoch aktiver Substanzen, wie z.B. Zytostatika oder auch bestimmter Antibiotika, diese Stoffe gezielter und dadurch geringer dosiert einsetzen zu können, als wenn diese über den üblichen Weg der oralen Darreichung eine systemische, den gesamten Organismus betreffenden Wirkung entwickeln. Zusammenfassend kann festgestellt werden, daß in der wissenschaftlichen Literatur gezeigt wird, daß Fibrinkleber sowohl für hydrophile als auch für lipophile Wirkstoffe ein gutes Potential als Trägersystem mit verzögertem Freisetzungsprofil besitzen.

Als parenterale Depotarzneiformen sind bekannt
- wässrige Suspensionen für schwerlösliche Wirkstoffe durch subkutane oder intramuskuläre Injektionen. Beispiel: Insulinpräparate (Wirkungsdauer 12-36 h). Kennzeichen ist das sehr aufwendige, aseptische Herstellungsverfahren für parenterale Suspensionen.
- ölige Lösungen, Suspensionen: Wirkstoff in Öl gelöst bzw. suspendiert, wodurch die Absorption des Wirkstoffs in der wässrigen Phase des Gewebes (und dadurch die therapeutische Wirkstofffreisetzung) bestimmt bzw. verlangsamt wird. Präparate zeigen sich teilweise durch eine sehr lange Wirkdauer aus (Wochen bis Monate).
- Emulsionen: Nach subkutaner oder intramuskuläre Injektion verteilen sich die Emulsionen im Gewebe und werden dort absorbiert. Derartige Systeme sind derzeit noch durch ein hohes Maß an Unverträglichkeiten charakterisiert.
- Hochviskose Lösungen, Suspensionen, Hydrokolloide (Polyvidon, Cellulosederivate) insbesondere für Protein- und Peptidwirkstoffe (gleichzeitig Schutzkolloidwirkung und Suspensionsstabilisatoren).
- Einsatz partikulärer Vehikel - Mikropartikel, Liposomen, Implantate. Mikropartikel und Liposomen erscheinen makroskopisch als Suspension bzw. Dispersion. Implantate sind hingegen Festkörüer und werden als solche angewandt. Erste Präparate dieser Art sind bereits in verschiedenen Ländern eingeführt. Neben den nichtabbaubaren Siliciumimplantaten, die nach Freigabe des eingebetten Wirkstoffs durch einen kleinen chirurgischen Eingriff wieder entfernt werden müssen, haben partikuläre Vehikel den Vorteil, daß sie bioabbaubar sind und damit nach ihrer hydrolytischen Spaltung vom Organismus selbst vollständig aus dem Gewebe entfernt werden. Zu beachten ist, daß die entstehenden Abbauprodukte weder toxisch noch immungen oder karzinogen sein dürfen. Die am meisten verwendeten Polymere sind daher aus gewebeverträglichen Milchsäure- bzw. Glykolsäuremonomeren (PLGA) hergestellt. Es sind jedoch auch Mikropartikel aus Albumin in der Radiodiagnostik bekannt, die sich insbesondere aufgrund deren guter Verträglichkeit und Bioabbaubarkeit gut eignen. Ähnliches kann auch für partikuläre Vehikel aus Fibrinkleber-Komponenten gelten.

Den technischen Lösungen zur Darstellung parenteraler Depotarzneiformen mittels gelöster, suspendierter oder emulgierter Wirkstoffe in Wasser, Lösungsmitel oder Öl ist gemeinsam, daß es sich dabei stets um sehr aufwendige Herstellungsverfahren und vor allem auch um teilweise komplizierte Applikationen handelt, deren pharmakologische Wirkung sich erst nach einer Reihe von (bio-)chemisch-physikalischen Absorptions-, Transport- bzw. Lösungsvorgängen im Organismus entfalten kann. Als Folge davon läßt sich die tatsächliche Bioverfügbarkeit nur eingeschränkt kontrollieren und verfolgen.

Eine vorteilhafte Entwicklung wäre es daher, für derartige (bisher flüssig zu verabreichende) Trägersysteme eine Darreichungsform zu finden, die durch eine definierte Matrix-Struktur, die während der Verabreichung für die Dauer der angestrebten Wirkstofffreisetzung konstant bleibt bzw. allmählich abgebaut wird, charakterisiert wird. Dies sind Eigenschaften, die z.B. bei bestimmten festen pharmazeutischen Darreichungsformen (wie Tabletten) bereits realisiert sind, die ein bestimmtes, retardiertes Freisetzungsverhalten z.B. im Magen-Darm-Trakt besitzen. Dies läßt sich nicht mit flüssigen Verabreichungen, wie diese für Fibrinkleber-Komponenten derzeit üblich sind, realisieren. Derartige, neue Darreichungsformen sollen verbesserte Depotarzneiformen sein.

Vorteilhaft erscheint dagegen der Einsatz partikulärer Vehikel, die u.U. direkt am Wirkort eingesetzt werden können, sofern diese dort geeignet fixiert werden können. Die Wirkstofffreisetzung erfolgt dann z.B. über diffusionsgesteuerte Absorption im Gewebe oder am Wirkort. Die Applikation könnte dabei vorteilhaft z.B. als trockenes Pulver direkt erfolgen, ohne daß die Mikropartikel in einer Trägerflüsigkeit suspendiert werden. Die direkte trockene Applikation durch Injektion der trockenen Partikel kann z.B. mit Hilfe der PowderJect® Technologie ("needle free Injection") erfolgen. Geeignete Partikelgrößen für dieses Verfahren liegen zwischen 50 und 200 µm. Auch ist es möglich, kleinere Partikel als Suspension zu injizieren oder größere Förmlinge mit einer dafür geeigneten, größeren Kanüle zu implantieren.

Hergestellt werden die Mikropartikel entweder durch Phasentrennverfahren oder durch Sprühtrocknung einer Polymer-Wirkstoff-Lösung bzw. -suspension. Es sind ebenfalls sterile oder zumindest aseptische Herstellungsbedingungen gefordert, die sehr anspruchsvoll sind. Umgangen werden diese Anforderung meist dadurch, daß die Mikropartikel nach der Sprühtrocknung sterilisiert werden. Dieses Vorgehen läßt sich jedoch nur für z.B. synthetische Polymere, die durch den Sterilisationsprozeß nicht unerwünscht verändert werden, anwenden. Thermisch labile, biologische Polymere mit spezifischer Aktivität auf Proteinbasis, wie z.B. Fibrinkleber bzw. Fibrinogen, können durch eine derartige Behandlung u.U. denaturiert werden, wodurch diese Verfahren für diese Art von Polymere nicht in Frage kommt. Weiterhin sind sprühgetrocknete Partikel vor allem auch dadurch gekennzeichnet, daß die Partikelgrößen sehr klein sind (in der Regel < 20 µm) und dadurch leicht zum Stauben neigen und praktisch nicht rieselfähig sind, wodurch eine exakte Dosierung bzw. direkte Applikation des pulverförmigen Feststoffs stark eingeschränkt wird.

In der wissenschaftlichen Literatur wird von Senderoff et al. bereits die Anwendung von Fibrinklebern als Trägersystem für therapeutisch wirksame Komponenten auf der Basis von Mikropartikeln beschrieben, wobei dort die Systeme Fibrinkleber als Mikropartikel, Fibrinkleberpartikel mit Zuckercoating und Fibrinkleberstreifen mit dispergierten Wirkstoffpartikeln vorgeschlagen werden. Nach vorhergehender Emulgierung des Wirkstoffs, Extraktion mit einem organischen Lösungsmittel und anschließender Verdampfung des Lösungsmittels können Mikropartikel gewonnen werden. Insbesondere dann, wenn diese Herstellungschritte unter aseptischen Bedingungen im industriellen Maßstab durchgeführt werden müssen, handelt es sich dadurch um ein sehr aufwendiges Verfahren, welches aus industrieller Sicht als sehr problematisch zu bewerten ist.

Somit stellt sich die Aufgabe, feste Partikel in geeigneter Weise herzustellen, daß diese als Trägersysteme für therapeutisch wirksame Substanzen eingesetzt werden können, und auch gleichzeitig als Feststoff verabreicht werden können. Bei den das Trägersystem bildenden Komponenten handelt es sich im wesentlichen um bioabbaubare Blutplasmaproteine, die ausgewählt sind aus Thrombin, Fibrinogen und Albumin oder deren Mischungen. Albumin, zeichnet sich dadurch aus, dass es im Organismus bereits eine wichtige, biochemische Transportfunktion besitzt und deshalb sowohl gut verträglich als auch abbaubar sind.

Erfindungsgemäß werden Partikel zur Verfügung gestellt die durch ein Granulationsverfahren in einer Wirbelschicht hergestellt worden sind, die die Komponenten Fibrinogen und Thrombin enthalten. Vorgegangen wird dabei analog zu dem in der Deutschen Anmeldung 198 49 589.7 beschriebenen Verfahren bei dem entweder Mischgranulate enthaltend mindestens Fibrinogen und Thrombin oder auch Granulat-Mischungen, die eine Mischung von Fibrinogen- und Thrombin-haltigen Granulaten darstellt mittels eines Wirbelschichtverfahrens hergestellt werden. Neben den Fibrinkleber-Komponenten sind weiterhin mindestens eine oder auch mehrere zusätzliche therapeutische, pharmakologische Wirkstoffkomponente(n) so auf die Partikel bzw. die Granulate aufgetragen werden, daß diese dort ausreichend fixiert werden, um eine Applikation zusammen mit den Fibrinkleber-Komponenten zu ermöglichen. Die Wirkstoffe bzw Kombinationen sind aus den Wirkstoffen nach Patentanspruch 1 ausgewählt. Dies kann bei Wirbelschichtanwendungen üblicherweise so erfolgen, daß der zusätzliche Wirkstoff zusammen mit mindestens einer der Fibrinkleber-Komponenten, bevorzugt mit Fibrinogen oder auch mit Thrombin gleichzeitig aus einer Polymer-Wirkstoff-Lösung oder -Suspension versprüht wird oder auch daß der Wirkstoff anschließend auf das sowohl Fibrinogen- als auch Thrombin-haltige Granulat aus einer Polymer-Wirkstoff-Lösung oder -Suspension aufgesprüht wird. Bei der ersten Variante kann der Wirkstoff z.B. direkt in das Granulatinnere in die feste Fibrinkleber-Matrix eingebunden werden, wobei bei der zweiten Variante der Wirkstoff bzw, die Wirkstoffe auf der Partikel- bzw. Granulatoberfläche gebunden wird. Weiterhin sind die für Wirbelschichtverfahren bekannten zusätzlichen Prozeßoptionen wie Aufbringen eines (Schutz-)Coatings bestehend aus einem Lack oder auch aus einem Kolloid (Polyvidon oder Cellulosederivate) möglich. Diese Coatings können z.B. innerhalb des Granulates als Trennschichten oder nur als reines äußeres Coating auf der Oberfläche angebracht werden. Zweck dieser Coatings kann sein, daß dadurch ein Schutz z.B. des Wirkstoffs erzeilt wird. (z.B. Coating mit Antioxidantien bei oxidationsempfindlichen Wirkstoffen) oder auch um die verzögerte Wirkstofffreisetzung noch zu verändern (verlängern). Zu beachten ist dabei jedoch, daß mögliche äußere Coatings vermutlich auch die adhäsive Wirkung des Fibrinklebers reduzieren oder unter Umständen auch verstärken können. Auch denkbar ist ein Coating aus Molekülen mit einer bestimmten Affinität zu bestimmten Geweben. Neben den Fibrinkleber-Komponenten als Trägerpolymer, können in analoger Verfahrensweise auch Granulate bzw. Pulver aus Albumin hergestellt werden.

Auch hier gilt, daß für Produkte, die als Parenteralia eingesetzt werden, sehr strenge Herstellungsvorschriften zu beachten sind, insbesondere hinsichtlich der konsequenten Ausrichtung aller Verfahrens- bzw. prozeßtechnischen Maßnahmen auf höchste mikrobiologische Reinheit. Sowohl die keimfreie (aseptische) Abfüllung insbesondere bei temperaturempfindlichen Zubereitungen, die nicht im Endbehälter sterilisiert werden können, als auch das Gebot niedriger Ausgangskeimzahlen zwingen hier zur Anwendung optimaler Sauberkeit und entsprechend entwickelter (aseptischer) Produktionslinien.

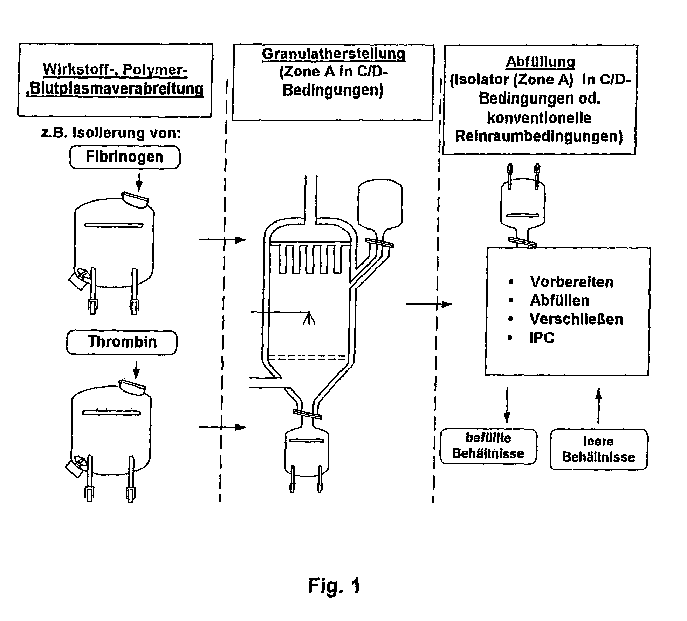

Vorgeschlagen wird, gemäß Fig. 1 die prinzipiellen Herstellungsschritte zur Isolierung bzw. Gewinnung der Polymere (z.B. die Blutplasmaproteinfraktionen Fibriogen und Thrombin) und der Wirkstoffe, sowie die Herstellung des Pulvers bzw. der Granulate bzw, der Mikropellets und die Abfüllung der Granulate in die Endverpackung zu trennen. Dabei können je nach den Anforderungen die jeweils von den nationalen und internationalen Richtlinien (z.B. GMP-Richtlinien) geforderten Raumklassen (Reinraum Zone A bzw. Raumzone C/D) entsprechend eingehalten werden.

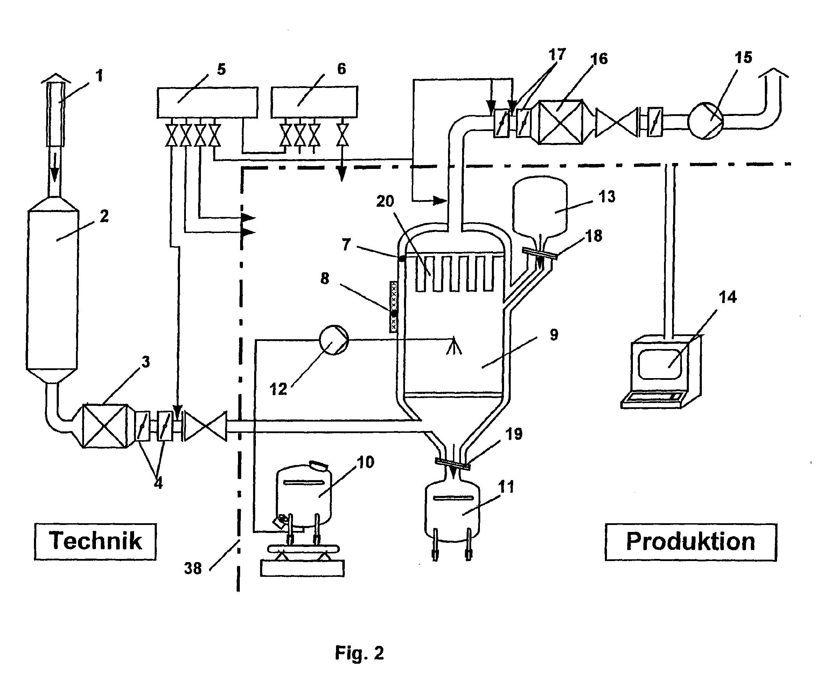

Für den Bereich der Granulat- bzw. Pulver- oder Pelletherstellung (Fig. 2) gilt -wie in der pharmazeutischen Produktion üblich -, daß der Produktions- und der Technikbereich räumlich klar, z.B. durch bauliche Maßnahmen 38, voneinander getrennt werden. Im Technikbereich befinden sich die notwendigen technischen Zusatzeinrichtungen, die für das Betreiben der Anlage erforderlich sind. Dies kann im Bereich der Ansaugung von Frischluft 1, die Prozeßluftaufbereitung, inkl verschiedener Filterstufen, Heizung und Kühlung, 2, einen zusätzlichen Sterilfilter 3, ein sterilisierbares Doppelklappensystem 4 und im Bereich der Abluft wiederum ein sterilisierbares Doppelklappensystem 17, einen Sterilfilter 16 und den Ventilator 15 umfassen. Ebenso im Technikbereich angeordnet sind eine Umlauf-Reinigungsstation 5 zur CIP (Cleaning-In-Place)-Reinigung der Anlage und verschiedener Anlagenkomponenten sowie ein Reinstdampferzeuger 6 zur Bereitstellung von Sterildampf zur Sterilisierung der Anlage, der Reinigungsanlage und weiterer im Detail noch zu spezifizierender Komponenten. Über die Reingiungsstation 5 kann die Anlage gemäß einem festzulegenden Reinigungsprogramm (Paarameter sind Art des bzw. der Reinigungsmittel, Reinigungszeiten, Temperaturen und Menge der Reinigungsmittel bzw. des zum Spülen bzw. Nachspülen verwendeten Wassers) gereinigt werden. Über ein Rohrleitungssystem werden an der Anlage und im Rohrleitungssystem verschiedene Reinigungsdüsen angefahren. Gleiches gilt für den Vorgang der Sterilisierung, bei dem sowohl die Anlage als auch verschiedene Positionen im Rohrleitungssystem mit Sattdampf beaufschlagt werden. Die Sterilisierung erfolgt gemäß einer einzustellenden Rezeptur nach entsprechenden Autoklavier-Bedingungen: nach dem Aufheizen der Anlage bis auf die gewünschte Sterilisationstemperatur (z.B. 121 °C und 3 bar Sattdampfdruck), wird eine bestimmte Haltezeit die gesamte Anlage und die zu sterilisierende Peripherie, z.B. 20 Minuten, eingehalten. Nach dem Abkühlen der Anlage auf z.B. Raumtemperatur oder einer höheren Prozeßtemperatur steht die dann sterilisierte Anlage für einen neuen Prozeß zur Verfügung, wobei darauf geachtet werden muß, daß die Anlage nicht geöffnet werden darf. Der Anlagenturm 9 besitzt neben speziellen CIP-fähigen Metallfiltern 20 weitere spezifische, CIP-fähige Konstruktionsdetails, wie z.B. spezielle, nicht dargestellte Aseptik-O-Ringe, und geeignete, kontaminationsfreie Andocksysteme 18, 19 für etwaige Vorlagebehälter 13 und Produktauffangbehälter 11, die ihrerseits allein z.B. dampfsterilisierbar sind. Die Behälterwand der Anlage ist als Doppelmantel 7, zum Beheizen und Kühlen, sowie u.U. mit einer zusätzlichen Isolierung 8 ausgeführt. Die Wirkstoff- bzw. Polymer-haltige Lösung oder Suspension wird über eine Pumpe 12, z.B eine Schlauchpumpe, aus einem geschlossenen Vorlagebehälter 10, der aus der Wirkstoff- bzw. Polyymerherstellung kommt, in die Anlage eingesprüht. Denkbar ist auch, daß der Vorlagebehälter 10 im Bereich der Poymer- bzw. Wirstoffherstellung stehen bleibt und über ein geeignetes Schlauch- bzw. Rohrleitungssystem mit der Sprühpumpe 12 verbunden wird. Die Bedienung der Anlage und der Peripherieeinrichtungen erfolgt über ein Bedienterminal 14, welches sowohl im Produktionsbereich als auch außerhalb des Produktionsbereiches installiert werden kann. Alternativ zu der beschriebenen offenen Herstellungsweise, bei der Frischluft über mehrere Filterstufen durch die Anlage gezogen wird und nach entsprechender Behandlung der Abluft wieder der Umgebung zugeführt wird, ist eine geschlossene Betriebsweise, bei der die Zuluft- und die Abluftseite mittels eines Kreislaufs verbunden sind, möglich.

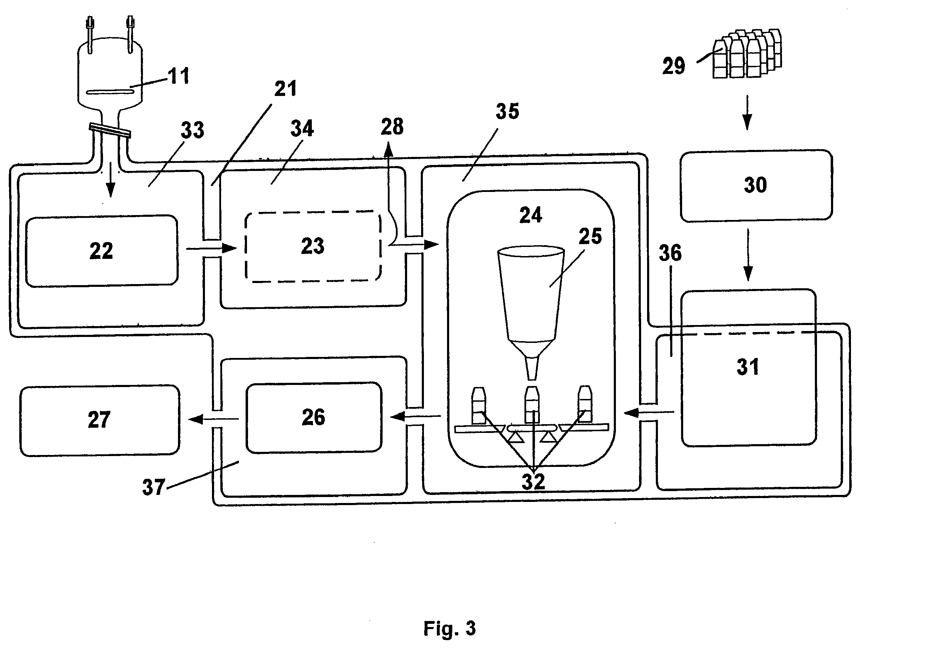

Die Abfüllung der Granulate bzw. der Pellets erfolgt wiederum in einem eigenen Bereich. Dieser Bereich kann z.B. in Isolatortechnik (Fig. 3) derart ausgeführt sein, daß der Produktauffangbehälter 11 geschlossen nach erfolgter Granulatherstellung an eine geeignete Isolator-Abfülleinheit 21 angedockt wird. Nach Öffnen der kontaminationsfreien Klappe 39 wird das Produkt in einem speziellen Bereich gesiebt 22 und u.U. gemischt 23. Bevor es in der eigentlichen Abfülleinheit 24 dann in die Einzelgebinde 32 dosiert wird, werden Muster gezogen und im Rahmen der vorgeschriebenen In-Prozeß-Kontrolle analysiert 28. Nachdem die Einzelgebinde 32 befüllt sind, werden die Behälter verschlossen 26 und verlassen so die Abfülleinheit und werden dann den nachfolgenden Produktionseinheiten, wie In-Prozeß-Kontrollen im Rahmen der Qualitätssicherung und -kontrolle und letztendlich der Verpackung bzw. Konfektionierung 27 zugeführt. In ähnlicher Weise werden der Abfülleinheit 24 die leeren Primärpackmittel 29, nachdem diese eine Wasch- und Spüleinrichtung 30 durchlaufen und in einem Sterilisationstunnel 31 sterilisiert werden, zugeführt. Innerhalb der Isolator-Abfülleinheit herrschen in den jeweiligen Behandlungsschritten mit offenem Produktehandling 33, 34, 35, 36 und 37 Reinraumbedingungen (d.h. Überdruck gegenüber der Umgebung, Partikelklasse 100 und Strömungsverhältnisse gemäß Laminar-Flow). Weiterhin besteht zwischen diesen Zonen 33, 34, 35, 36 und 37 ein definiertes Druckgefälle, so daß es zu keiner Kreuz-Kontamination kommen kann. Bei entsprechender baulichen Konzeption ist es auch möglich, auf die Übertragung des Produktes mittels des Auffangbehälters 11 zu verzichten. Dies sieht dann so aus, daß in einer mehrgeschößigen, vertikalen Anordnung die Abfülleinheit direkt unter dem Anlagenturm der Granulatherstellung angebracht wird. Auch muß jedoch dann die Granulatherstellung von der Abfülleinheit durch bauliche Maßnahmen getrennt werden.

Mögliche Formulierungen können sein:
- Mischungen von Partikeln bzw. Granulaten, die Fibrinkleberkomponenten und Wirkstoffen, die sowohl hydrophile, amphiphile oder auch lipophile Eigenschaften haben können. Die Wirkstofffreisetzung kann dabei auch durch aus dem Stand der Technik bekannte Formulierungszusatzstoffe beeinflußt werden. Weiterhin können Hilfsstoffe wie z.B. Lecithine (Ei- oder Sojalecithin) oder auch Tween zur Verbesserung der Benetzbarkeit den Partikeln bzw. den Granulaten zugemischt werden. Das Größenspektrum derartiger Pulver- bzw. Granulatmischungen kann in einem Bereich von 50 bis 500 µm oder bevorzugt in einem Bereich von 50 bis 200 µm oder auch von 200 bis 500 µm liegen.
- Mischungen von Partikeln bzw. Granulaten, die eingebunden z.B. Polymer-Mikropartikel mit zusätzlichen Wirkstoffen enthalten. Bei den Polymeren kann es sich auch um nicht Fibrinogen- bzw. Thrombin-haltige Polymere synthetischen Ursprungs, die jedoch ebenfalls biologisch abbaubar sind, handeln. Als Beispiel sind zu nennen Polymere aus Milchsäure/Glycolsäure (PLGA) oder Polyanhydride oder Polyorthoester oder andere gemäß dem Stand der Technik bekannte und geeignete Polymere. Es können jedoch auch Polymere auf Proteinbasis, wie z.B. Albumin, sein. Auch geeignet sind synthetische Polymere, die als synthetischer Fibrinkleber eingesetzt werden, wie z.B. Poly-Octylcyanoacrylat. Das Größenspektrum derartiger Pulver- bzw. Granulatmischungen kann in einem Bereich von 50 bis 500 µm oder bevorzugt in einem Bereich von 50 bis 200 µm oder auch von 200 bis 500 µm liegen.
- Mischungen von Partikeln bzw. Granulaten, die sich jeweils aus einem inneren Kern und einer äußeren Schicht zusammensetzen. Bei der äußeren Schicht handelt es sich um Fibrinkleber-Komponenten, um eine ausreichende Fixierung der Partikel bzw. der Granulate im Gewebe zu gewährleisten. Der innere Kern kann dagegen auch aus inerten, üblichen Hilfsstoffen, wie z.B. Kohlenhydrate (Lactose, Mannitol, etc.) gebildet werden. Durch entsprechende Auswahl der Hilfsstoffe bzw. durch entsprechende galenische Formulierung kann dann die Löslichkeit des Kern so beeinflußt werden, daß dieser z.B. nur schwer löslich ist und dadurch auch eine verzögerte bzw. verlängerte Freisetzung von Wirkstoffen bewirkt. Sowohl in die äußere Schicht als auch in den inneren Kern kann der zusätzliche Wirkstoff bzw. können die Wirkstoffe eingebunden sein. In Frage kommen dafür z.B. quervernetzte Polymere, z.B. Cellulosederivate, die z.B. bei Matrix-Tabletten zum Einsatz kommen. Das Größenspektrum derartiger Pulver- bzw. Granulatmischungen kann in einem Bereich von 50 bis 500 µm oder bevorzugt in einem Bereich von 50 bis 200 µm oder auch von 200 bis 500 µm liegen.
- Festkörper, die durch Verpressen der Partikel- bzw. der Granulatmischung zu Tabletten oder auch durch Komprimieren zu Komprimaten (z.B. Walzenkompaktierung mit anschließender Siebung (= Kalibrierung) zur Einstellung definierter Größen oder auch Brikettierung) hergestellt werden können. Dadurch ergeben sich weitere Freiheitsgrade für die galenische Formulierung, wodurch sich zusätzlich die Freisetzung verändern kann aber auch neue Applikationsformen möglich sein werden. Gemäß dem Stand der Technik sind z.B. folgende Modifikationen denkbar: Mantel-Kern-Tabletten, Matrixtabletten, Oros-Tabletten, Komponenten, die die Freisetzung steuern, Tablettengröße und -form. Mittels Kompaktoren lassen sich dagegen z.B. längliche, dünne Streifen aus dem Pulver bzw. dem Granulat herstellen, die wiederum direkt in eine Körperöffnung oder einen Schnitt nach chirurgischen Eingriffen flächig eingelegt werden könnte. Sowohl mittels Tabletten als auch mittels Extrudaten lassen sich definierte und gezielte Matrix-Strukturen der Fibrinkleber-Komponenten und der Wirkstoffkomponenten aufbauen. Auch denkbar sind z.B. zusätzliche stabilisierende, netzartige Gewebestrukturen, die entweder zusätzlich zusammen mit den Tabletten oder mit den Komprimaten appliziert werden können, wodurch der Einsatz zur Wundheilung bei chronischen Erkrankungen gegeben ist. Dadurch sind Anwendungen für den Gewebeersatz, z.B. Haut, möglich.
- kompakte, homogene Mikropellets mit einem mittleren Partikeldurchmesser von rund 50 µm. Dabei kann es sich um bioabbaubare, auch nicht Fibrinogen- oder Thrombin-haltige Mikropellets (z.B. Albumin, PLGA) handeln, die auf der äußeren Oberfläche mit einer Fibrinkleber-Komponenten-Schicht überzogen werden. Dadurch werden die bekannt guten Adhäsionseigenschaften des Fibrinklebers ausgenutzt. In dem inneren bioabbaubaren Polymer-Kern ist zusätzlich noch mindestens eine oder auch mehrere therapeutisch wirksame Substanz eingebaut. Vorteil ist, daß dadurch die Mikropellets besser lokal verabreicht werden und auch fixiert werden können. Auch können die Schichten einen unterschiedlichen zeitlichen Ablauf der Bioabbaubarkeit haben.
- kompakte, homogene Mikropellets mit einem mittleren Partikeldurchmesser von rund 50 µm, die aus einem Kern von Fibrinkleber-Komponenten bestehen und in die schwer-lösliche Wirkstoffe eingelagert sind.
- Poröse keramische Granulate oder Granulate aus Werkstoffen für den Knochenersatz, wie z.B. Calciumphosphate, die mit Blutplasmaproteinen beschichtet sind und wobei diese beschichteten Granulate zu einem Festkörper verpreßt werden. Dieser Festkörper kann dann als Knochenersatz eingesetzt werden. Hierbei ist es auch möglich, die Granulate noch mit Wirkstoffen wie Antibiotika oder Wachstumsfaktoren, wie z.B. BMP oder TGF-B-Type zu versetzen. Ein Beispiel für BMP ist Collagen. Dies kann so durchgeführt werden, daß in einem 1. Schritt die keramischen Granulate mit einer 1. fibrinogenhaltigen Schicht beschichtet werden. In einem 2. Schritt wird dann Thrombin mit z.B. Wachstumsfaktoren in die Wirbelschicht aufgetragen. Dies kann gemäß der DE 198 49 589 C1 erfolgen. Auf den Offenbarungsgehalt wird Bezug genommen.

Applikationsmöglichkeiten des Fibrinklebers als Trägersystem sind:
- Topische Anwendung in gleicher Weise wie herkömmlicher Fibrinkleber zur Blutstillung bei Wunden, chirurgischen Eingriffen, offenen Körperhöhlen oder über mucosale Membranen, z.B. Mund, Nase, Kolon oder Vagina, für lokale oder systemische Applikation.
- Anwendung als parenterale Depotarzneiform in Verbindung mit den Eigenschaften der Verträglichkeit, Adhäsivität, Bioabbaubarkeit in der Form als Pulver bzw. als Granulatmischung oder auch Mikropellets, ohne daß diese vor der Applikation gelöst oder suspendiert werden (z.B. mittels spezieller, Ject® Injektion, d.h. needle-free Injektion von Feststoffen).
- Anwendung als parenterale Depotarzneiform in Verbindung mit den Eigenschaften der Verträglichkeit, Adhäsivität, Bioabbaubarkeit als Mikropellets-Suspension bzw. einer Trägerflüssigkeit als Dispersion. In Frage kommen dafür z.B. ölige Suspensionen (Tri-Glyceride, Sesamöl). Um ein vorzeitiges Koagulieren der suspendierten Mikropellets vor bzw. bei der Injektion zu verhindern, können der Suspension Antikoagulatien (z:B. tri-NatriumCitrat) zugegeben werden. Bei den Polymeren auf der Basis der Fibrinkleber-Komponenten muß besonders auf die üblichen Probleme der derzeit verfügbaren Systeme für Fibrinkleber geachtet werden (= spontane Gerinnung durch vorzeitiger Kontakt der aktiven Komponenten Fibrinogen und Thrombin muß verhindert werden.
- Anwendung als Trägersystem für therapeutische Wirkstoffe über die nicht-parenterale Verabreichung (z.B. orale, topische, rektale oder vaginale Applikation).
- transdermale Applikation (Pflaster).
- Anwendung als Implantat,z.B. als Knocheneratz oder als Gewebe.

Das Verfahren zur Herstellung von Pulver oder Granulaten kann so ausgeführt werden, daß das Fluidisationsgas durch die Wirbelschichtkammer von unten nach oben geführt wird und die zu trocknende Flüssigkeit (Lösung oder Suspension) von oben (Top-Spray), von unten (Bottom-Spray) oder auch seitlich (Rotor-Wirbelschicht) über ein Sprühsystem eingesprüht wird. Das Fluidisationsgas hat gleichzeitig die Aufgabe, in der Wirbelkammer vorliegendes Produkt zu verwirbeln, die zum Verdunsten der Sprühflüssigkeit (Wasser oder organisches Lösungsmittel) benötigte Wärme dem Sprühstrahl oder dem feuchten Produkt zuzuführen und gleichzeitig die verdunstete Flüssigkeitsmenge aufzunehmen und abzutransportieren. Der Austrag des getrockneten Produktes wird einerseits durch die Wahl einer geeigneten Fluidisationsgeschwindigkeit (kleiner als die rechnerisch und experimentell ermittelbare sog. Austraggeschwindigkeit für das Produkt), andererseits auch durch einen im oberen Bereich der Wirbelkammer vorhandenen und regelmäßig abreinigbaren Produktrückhaltefilter oder auch durch einen anderen aus dem Stand der Technik bekannten Produktabscheider (wie z.B. ein Zyklonabscheider) verhindert.

Die im Sprühkegel fein zerstäubten Flüssigkeitströpfchen treffen dabei auf das aufgewirbelte pulverige Trägermaterial und trocknen dort aufgrund der für Wirbelschichtverfahren idealen Wärme- und Stoffübergangsverhältnisse, die im wesentlich eine Folge der sehr großen spezifischen Partikeloberflächen des verwirbelten Produktes sind. Während des Sprühens kommt es z.B. aufgrund der im Partikel langsam zunehmenden Produktfeuchte zur Ausbildung von Agglomeraten oder Granulaten und dadurch zu einer Zunahme der Partikelgröße.

Bei der Wahl der Prozeßbedingungen muß für thermisch labile Produkte (Polymere bzw. Wirkstoffe) primär darauf geachtet werden, daß diese durch hohe Temperaturen nicht geschädigt werden. Dies gilt insbesondere dann, wenn native Polymere auf Proteinbasis verarbeitet werden, besonders für die nativen Fibrinkleber-Komponenten (vorallem für Fibrinogen). Geeignete Zulufttemperaturen liegen z.B. zwischen 15 und 100 °C für die Produkttemperatur bevorzugt jedoch kleiner 37 °C (dies gilt besonders für Fibrinogen). Bei Albumin können dagegen auch Produkttemperaturen z.B. bis zu 50 °C möglich werden, ohne daß es zu einer Denaturierung des Albumins kommt. Berücksichtigt werden muß dabei, daß eine mögliche Inaktivierung immer im Zusammenhang mit einer bestimmten Feuchte betrachtet werden muß, d.h. die Temperaturstabilität nimmt mit abnehmender Produktfeuchte im Feststoff zu, so daß gegen Ende der Trocknung auch höhere Temperaturen akzeptabel sein können.

Ein weiterer wichtiger Parameter zur Bewertung eines Prozesses ist die sog. Ausbeute oder auch Wiederfindungsrate der eingesprühten Substanz auf dem Trägermaterial. Ziel ist natürlich, nahezu 100 % der über die Sprühflüssigkeit eingebrachten Substanz auf dem Träger oder in der Wirbelkammer wiederzufinden. Auch hier gilt, daß die Parameter (z.B. Fluidisationsgeschwindigkeit, Menge Produktvorlage, Position der Spühdüse, Apparategröße- und geometrien) gemäß dem bekannten Stand der Technik der Wirbelschichttechnik geeignet ausgewählt und über Versuche angepaßt werden müssen und können.

Die Trocknung muß bis zu einer Restfeuchte erfolgen, die so klein ist, daß je nach den gewählten Lagerbedingungen keine unerwünschten molekularen Änderungen der Polymere beobachtet werden oder daß es bereits zu einem merklichen Wirkstoffververlust kommt. Für die Polymermatrix auf der Basis der Fibrinkleber-Komponenten muß die Restfeuchte so gering sein, daß die Gerinnungsreaktion nicht spontan abläuft. Geeignete Lagerbedingungen können sein: Kühllagerung bei 4 bis 8 °C oder Raumbedingungen (20 °C). Das Granulat kann zusätzlich in einer schützenden Atmosphäre (z.B.

Stickstoff oder Kohlendioxid) und z.B. unter Lichtausschluß eingeschlossen sein. Mögliche Restfeuchten können dann z.B. zwischen 0,1 - 5 % Wassergehalt liegen.

Homogene, wirkstoffhaltige Mikropellets, deren Polymere mittels Fibrinkleber-Komponenten oder anderer bioabbaubarer Protein oder auch mittels bioabbaubarer Polymere, wie z.B. Milchsäure/Glykolsäure-Ploymere, gebildet werden, können dagegen bevorzugt auch durch direktes Einsprühen aus einer Polymer-Wirkstoff-Lösung oder -Suspenion in eine leere Anlage erzeugt werden. Dabei werden in der Anlage in-situ Granulatkeime bzw. fein verteilte Partikel erzeugt, die als Starterkerne für eine weitere Granulation dienen können. Die dafür zu verwendende Anlage kann z.B. ein Sprühturm oder auch eine Wirbelschichtanlage mit ausreichend freier Flugstrecke für die versprühten Flüssigkeitströpfchen sein. Bei Einhaltung geeigneter Prozeßbedingungen können die versprühten Flüssigkeitströpfchen entsprechend den Verhältnissen eines Sprühtrockners (jedoch mit reduzierten Trocknungstemperaturen) in einer Wirbelschichtanlage getrocknet werden, bevor sie z.B. im noch feuchten Zustand die Behälterwand berühren und dort kleben bleiben. Diese so erzeugten feinen Partikel werden durch das Fluidisationsgas in Bewegung und in der Schwebe gehalten und kommen so mit dem Sprühnebel der weiterhin eingesprühten Flüssigkeit in Kontakt und beginnen dann zu granulieren. Auf diese Weise kann, insbesondere durch sehr vorsichtige Fahrweise des Prozesses während des Anfahrens des Prozesses, in der ursprünglich leeren Anlage ein definiertes Granulatwachstum generiert werden. Dies kann z.B. durch Zugabe bekannter Bindemittel unterstützt werden. Durch Kombination mit einem klassierenden Granulataustrag (z.B. über einen Zick-Zack-Sichter und klassierendem Luftstrom) besteht die Möglichkeit, Granulat mit einer definierten Partikelgröße in der Anlage zu erzeugen und den Prozeß sogar in einer kontinuierlichen oder quasikontinuierlichen Fahrweise zu betreiben. Das hier beschriebene Verfahren basiert im wesentlich auf dem Europäischen Patent EP 85103501.4.

Damit lassen sich die folgenden physikalischen Produkteigenschaften erzielen:
- Partikeldichte: 250 - 2000 g / ml, bevorzugt 500 - 1500 g / ml
- Parikelgrößen: 20 - 1000 µm, bevorzugt 50 - 500 µm oder 30 - 350 µm
- Partikelgrößenverteilung: z.B. Über- bzw. Unterkorn +/- 50 % von der mittleren Korngröße, bevorzugt +/ - 25 % der mittleren Korngröße
- Wirkstoffgehalt: 0,1 - 100 %
- Produkteigenschaften: staubfrei, rund, schalenförmiger Partikelaufbau, vergleichsweise hohe Dichte, keine inerten Kerne, kein Abrieb.

Die verwendeten therapeutischen Wirkstoffe sind den folgenden Wirkstoffklassen zuzuordnen :

### Human-Anwendungen:

- Cortico-Steroide
- Antimykotika
- Neuroleptika
- Antiepileptika
- Steroidhormone
- Krebshemmende Hormone
- Zytostatika
- Narkotika, Analgetika
- Peptidhormone (Supstitutionstheapie)
- Antirheumatika
- Impfstoffe, Antikörper
- Monoklonale Antikörper

### Tiermedizin:

- Hormone
- Insektizide, Anthelminika
- Impfstoffe, Antikörper

## Patentansprüche

1. Depotarzneimittelformulierung in Form eines Mischgranulats bestehend aus einem Träger in Form eines mikroporösen Granulates mit einer Korngröße von 20 bis 500 *µ*m, hergestellt aus mittels Wirbelechichttrocknung unter Erhalt ihrer Eigenschaften getrockneter bioabbaubarer Blutplasmaproteine ausgewählt aus Thrombin, Fibrinogen, Albumin oder deren Mischungen und einen als Depot zu applizierenden Wirkstoff oder eine Wirkstoffkombination ausgewählt aus, Corticosteroide, Antimykotika, Neuroleptika, Antiepileptika, Steroidhormone, krebshemmende Hormone, Zytostatika, Narkotika, Analgetika, Peptidhormone Antirheumatika, Impfstoffe, Antikörper, monoklonale Antikörper.

2. Depotarzneimittelformulierung nach Anspruch 1, **dadurch gekennzeichnet, daß** der Wirkstoff in das Granulatinnere eingebunden ist.

3. Depotarzneimittelformulierung nach Anspruch 1, **dadurch gekennzeichnet, daß** der Wirkstoff auf der Granulatoberfläche gebunden ist.

4. Depotarzneimittelformulierung nach einem der Ansprüche 1 bis 3, **dadurch gekennzeichnet, daß** diese ein Festkörper ist, der durch Verpressen der Granulate hergestellt worden ist.

5. Depotarzneimittelformulierung nach einem der Ansprüche 1 bis 3, **dadurch gekennzeichnet, daß** sie in Form von kompakten homogenen Mikropellets mit einem mittleren Partikeldurchmesser von 35 bis 500 *µ*m, bevorzugt 50 bis 150 *µ*m, vorliegt.

## Claims

1. Depot drug formulation in the form of a mixed granulate consisting of an excipient in the form of a microporous granulate with a particle size of between 20 and 500 µm made from biodegradable blood plasma proteins that are dried using the fluid bed combustion process while retaining their properties, and that are selected from thrombin, fibrinogen, albumen or mixtures thereof, and an active agent or combination of active agents for application in the form of a depot and selected from corticosteroids, antimycotics, neuroleptics, anti-epileptic agents, steroid hormones, cancer-suppressing hormones, cytostatics, narcotics, analgesics, peptide hormones, anti-rheumatics, vaccines, antibodies, monoclonal antibodies.

2. Depot drug formulation as in claim 1,
**characterised in that**
the active agent is bonded into the interior of the granulate.

3. Depot drug formulation as in claim 1,
**characterised in that**
the active agent is bonded on to the surface of the granulate.

4. Depot drug formulation as in one of claims 1 to 3,
**characterised in that**
it is a solid which is produced by compressing the granulates.

5. Depot drug formulation as in one of claims 1 to 3,
**characterised in that**
it is in the form of compact homogeneous micropellets with an average particle diameter of between 35 and 500 µm, preferably between 50 and 150 µm.

## Revendications

1. Formulation de médicament à libération prolongée sous la forme de granulés mixtes, constituée d'un véhicule sous la forme de granulés microporeux ayant une granulométrie de 20 à 500 *µ*m, fabriqués à partir de protéines de plasma sanguin biodégradables séchées par séchage en couche fluidisée en conservant leurs propriétés, les protéines étant choisies parmi la thrombine, le fibrinogène, l'albumine ou leurs mélanges et constituée d'un principe actif à appliquer comme substance ou combinaison de principes actifs, à libération prolongée choisie parmi les corticostéroïdes, les antimycosiques, les neuroleptiques, les antiépileptiques, les hormones stéroïdiennes, les hormones anticancéreuses, les cytostatiques, les narcotiques, les analgésiques, les hormones peptidiques, les antirhumatismaux, les vaccins, les anticorps et les anticorps monoclonaux.

2. Formulation de médicament à libération prolongée selon la revendication 1, **caractérisée en ce que** le principe actif est intégré à l'intérieur des granulés.

3. Formulation de médicament à libération prolongée selon la revendication 1, **caractérisée en ce que** le principe actif est lié sur la surface des granulés.

4. Formulation de médicament à libération prolongée selon l'une quelconque des revendications 1 à 3, **caractérisée en ce qu'**elle constitue un corps solide fabriqué par compression des granulés.

5. Formulation de médicament à libération prolongée selon l'une quelconque des revendications 1 à 3, **caractérisée en ce qu'**elle se présente sous la forme de micropastilles compactes homogènes ayant un granulométrie moyenne de 35 à 500 *µ*m, de préférence de 50 à 150 *µ*m.
